# EUROPEAN PATENT APPLICATION

(11) **EP 1 977 761 A2**
(43) Date of publication of application: **08.10.2008**
(21) Application number: 06846929.5
(22) Date of filing: 28.12.2006
(51) Int. Cl.: A61K 39/095, C12Q 1/68, A61P 31/04

(54) **PHARMACEUTICAL COMPOSITIONS CONTAINING PROTEIN NMA0939**

(30) Priority: 29.12.2005 CU 5027905
(71) Applicant: CENTRO DE INGENIERIA GENETICA Y BIOTECNOLOGIA (CIGB), Ciudad de la Habana 10600 (CU)
(72) Inventor: PAJÓN FEYT, Rolando, La Habana 32400 (CU); SARDIÑAS GARCIA, Gretel, Ciudad De La Habana 10500 (CU); LAGE CASTELLANOS, Agustín, Ciudad De La Habana 10600 (CU); YERO CORONA, Daniel, Ciudad De La Habana 10300 (CU); GARCIA DIAZ, Darién, Ciudad De La Habana 11300 (CU); GONZALEZ BLANCO, Sonia, Ciudad De La Habana 12100 (CU)
(74) Representative: Hatzmann, Martin
(86) International application number: PCT/CU2006/000019
(87) International publication number: WO 2007/073705

(57) **Abstract**

The present invention is related to field of medicine, particularly to the development of pharmaceutical formulations containing NMA0939 protein. Formulations described in this invention are able to confer protection against different diseases caused or not by pathogenic agents. NMA0939 protein was identified as a *Neisseria meningitidis* outer membrane vesicle component, and it was obtained through recombinant DNA technology being its immunogenicity and protective activity evaluated in animal models. Due to the high level of conservation the NMA0939 coding gene has shown, pharmaceutical compositions containing this protein have a high value as inducers of a cross-reactive immune response. Formulations presented in this invention are applicable to the field of human medicine.

## Description

### Technical Field

The present invention is related to field of medicine, particularly to the development of new vaccine formulations of preventive or therapeutic application, that allow an increase in the quality of immune response against vaccine antigens of diseases from different sources.

### Previous state of the Art

*Neisseria meningitidis,* a Gram-negative diplococcus who's only know host is man, is the causal agent of meningococcal meningitis. Usually this bacterium is found in asymptomatic carriers among the normal population, being this niche the most common source for its microbiological isolation.

On world basis, small children less than two years of age are the more susceptible population for contracting meningococcal meningitis, however, young adults and normal adult population may also be affected.

Untreated meningococcal disease has a fatal course for most affected individuals, and vaccination could prevent this situation, by halting the events as early as at the bacterial colonization phase.

Several strategies have been developed with the aim of obtaining a vaccine able to fulfill the needed requirements in order to induce protection against this disease in general population. For this purpose capsular antigens have been taken into account, since their immunological specificity has allowed the classification into serogroups of this microorganism. Five of these serogroups have been defined as responsible of most of the clinical cases of meningococcal disease all around the world. Serogroup A is the principal cause of epidemics in sub-Saharan Africa. Serogroups B and C are associated, in most cases, to the occurrences in developed nations. Serogroups Y and W135 are common in most of the recurrent cases of the disease, and they are prevalent in some areas of USA, with a marked increase in last few years. From this data it is obvious the reason of the use, study, and evaluation of capsular polysaccharides as vaccine candidates. A tetravalent vaccine, based on capsular polysaccharides, conferring protection against serogroups A, C, Y, and W-135 has been licensed in Unite States. Elicited antibodies after vaccination are serogroup-specific (Rosenstein N. et al. 2001. Meningococcal disease. N. Engl. J. Med, 344, 1378-1388).

Serogroup B, which is different from the rest, continues to be a significant cause of endemic and epidemic meningococcal disease, and thi is mainly due to the complete lack of efficient vaccines against it. It has been noted that B capsular polysaccharide is poorly immunogenic, plus the existence of the theoretical risk for a vaccine based on this compound to induce immuno-tolerance and autoimmunity because of its structural similarity to oligosaccharide chains that are present in human neural fetal structures. (Finne J. et al. 1987. An IgG monoclonal antibody to group B meningococci cross-reacts with developmentally regulated polysialic acid units of glycoproteins in neural and extraneural tissues. J. Immunol, 138: 4402-4407). Therefore, the development of vaccines against serogroups B is concentrated in the use of sub-capsular antigens.

### Outer membrane proteins and vesicle vaccines

Initial attempts, in the 70s, to produce vaccines based on outer membrane proteins were based on the LPS depletion of outer membrane protein preparations by detergent (Frasch CE and Robbins JD. 1978. Protection against group B meningococcal disease. III. Immunogenicity of serotype 2 vaccines and specificity of protection in a guinea pig model. J Exp Med 147(3):629-44). The outer membrane proteins, OMPs, were then precipitated to produce aggregates suspended in sodium chloride. Despite promising results in animal studies, these vaccines failed to induce bactericidal antibody in either adults or children (Zollinger WD, et al. 1978. Safety and immunogenicity of a Neisseria meningitidis type 2 protein vaccine in animals and humans. J. Infect. Dis. 137(6):728-39), the poor performance of these vaccines was largely attributed to the loss of tertiary structure that accompanied precipitation. The next logical step was, therefore, to produce a vaccine with proteins displayed in their native conformation in the form of vesicles of outer membrane (Zollinger WD, et al. 1979. complex of meningococcal group B polysaccharide and type 2 outer membrane protein immunogenic in man. J. Clin. Invest. 63(5):836-48, Wang LY and Frasch CE. 1984. Development of a Neisseria meningitidis group B serotype 2b protein vaccine and evaluation in a mouse model. Infect Immun. 46(2):408-14136).

These outer membrane vesicle vaccines were significantly more immunogenic than the OMP aggregates and immunogenicity was shown to be further enhanced by adsorption to the adjuvant aluminium hydroxide (Wang LY and Frasch CE. 1984. Neisseria meningitidis group B serotype 2b protein vaccine and evaluation in a mouse model. Infect Immun. 46(2):408-14136).

A number of efficacy trials have been carried out using soluble outer membrane vesicle vaccines of different formulations. The two vaccines most extensively studied were developed in the 1980s in response to outbreaks of disease in Cuba (Sierra GV et al. 1991. Vaccine against group B Neisseria meningitidis: protection trial and mass vaccination results in Cuba. NIPH Ann Dis. 14(2):195-210) and Norway (Bjune G, et al. 1991. Effect of outer membrane vesicle vaccine against group B meningococcal disease in Norway. Lancet. 338(8775):1093-6), respectively. The OMV vaccine produced by the Finlay Institute in Cuba (commercially marketed as VA-MENGOC-BC ) is produced from strain B:4:P1.19,15 with serogroup C polysaccharide and a preparation of high molecular weight OMPs and is adsorbed to aluminium hydroxide (Sierra GV et al. 1991. Vaccine against group B Neisseria meningitidis: protection trial and mass vaccination results in Cuba. NIPH Ann Dis. 14(2):195-210). This vaccine contributed to the rapid decline of the epidemic in Cuba (Rodriguez AP, et al. The epidemiological impact of antimeningococcal B vaccination in Cuba. 1999. Mem Inst Oswaldo Cruz. 94(4):433-40).

The vaccine produced by the Norwegian National Institute for Public Health (NIPH) was similarly intended initially for use during a period of hyper endemic disease caused by another organism from the ET-5 clone (B:15:P1.7,16). It was also a monovalent vaccine produced from purified outer membrane vesicles adsorbed onto aluminium hydroxide (BjuneG, et al. 1991. Effect of outer membrane vesicle vaccine against group B meningococcal disease in Norway. Lancet. 338(8775):1093-6).

Outer membrane vesicle vaccines appear to effectively present outer membrane proteins in a sufficiently natural conformation to allow the generation of functional bactericidal antibodies, at least in teenagers and adults. The antibody responses generated have also been shown to increase opsonophagocytosis of meningococci. The precise formulation of the vaccines (i.e. OMP content, LPS content and the presence or absence of adjuvant) have a significant impact on immunogenicity (Lehmann AK, et al. 1991. Immunization against serogroup B meningococci. Opsonin response in vaccinees as measured by chemiluminescence. APMIS. 99(8):769-72, Gomez JA, et al. 1998. Effect of adjuvants in the isotypes and bactericidal activity of antibodies against the transferrin-binding proteins of Neisseria meningitidis. Vaccine. 16(17):1633-9, Steeghs L, et al. 1999. Immunogenicity of Outer Membrane Proteins in a Lipopolysaccharide-Deficient Mutant of Neisseria meningitidis: Influence of Adjuvants on the Immune Response. Infect Immun. 67(10):4988-93).

The antigenic profile of disease isolates also changes rapidly and a vaccine with coverage of only a limited number of selected strains is likely to become ineffective within a few years unless the vaccine composition is changed to mirror local epidemiology.

At present OMV vaccines have been used more widely than any other serogroup B vaccine and are potentially useful in the context of outbreaks of disease caused by a single PorA type.

The immunogens that generate cross-reactivity between strains have yet to be fully defined. Studies of post-vaccination sera from both Finlay Institute and NIPH vaccine trials suggested that antibodies against both PorA (P1, the class 1 serosubtype protein) and OpcA (another major OMP, formerly known as Opc) (Wedege E, et al. 1998. Immune Responses against Major Outer Membrane Antigens of Neisseria meningitidis in Vaccinees and Controls Who Contracted Meningococcal Disease during the Norwegian Serogroup B Protection Trial. Infect Immun. 66(7): 3223-31), were both important in the mediation of serum bactericidal activity (with PorA more immunogenic) both these antigens show marked strain to strain variability.

The prominence of PorA protein, and the significant level of variability in this protein which appears to undergo continuous variation both between and during outbreaks in epitopes to which most of the bactericidal activity in post-vaccination (and post-disease) is directed enhanced concerns that protection offered by single strain (monovalent) OMV-based vaccines might be serosubtype restricted (Jelfs J, et al. 2000. Sequence Variation in the porA Gene of a Clone of Neisseria meningitidis during Epidemic Spread. Clin Diagn Lab Immunol. 7(3):390-5).

In an attempt to overcome this potential problem, an OMV vaccine was developed in The Netherlands at RIVM that contained PorA proteins from six different prevalent pathogenic isolates (Van Der Ley P and Poolman JT. 1992. Construction of a multivalent meningococcal vaccine strain based on the class 1 outer membrane protein. Infect Immun. 60(8):3156-61, Claassen I, et al. 1996. Production, characterization and control of a Neisseria meningitidis hexavalent class 1 outer membrane protein containing vesicle vaccine. Vaccine. 14(10):1001-8). In this case the vaccine vesicles were extracted from two variants of the well-characterized H44/76 strain which had been genetically engineered lo express three separate PorA proteins.

### The search for a universal antigen

It is clear that outer membrane proteins (OMP) can induce a functional immune response against serogroup B disease but that none of the vaccines so far developed are universally protective due to the great heterogeneity of the surface exposed regions of the outer membrane proteins. The modest cross-reactive immunity induced by the outer membrane vesicles (OMV) vaccines has fuelled the search for an outer membrane antigen (or group of antigens), which induces functional antibodies and which is present on all meningococcal strains. Such antigens, if they were present on all strains irrespective of serogroup, might form the basis of a truly universal meningococcal vaccine, which would eliminate the potential problem of capsular switching on pathogenic strains following polysaccharide vaccination.

Once it became apparent that the variability of the immunodominant PorA protein would limit its use as a universal vaccine, a number of the other major outer membrane proteins were considered for their vaccine potential and several of these are under further development. Those which have been considered include class 5 proteins (OpcA), NspA and iron regulated proteins (TbpA and B, FbpA, FetA). TbpB forms part of the transferrin binding complex with TbpA. Recent work suggests that TbpA has both a greater functional role in iron binding (Pintor M, et al. 1998. Analysis of TbpA and TbpB functionality in defective mutants of Neisseria meningitidis. J Med. Microbiol. 47(9): 757-60) and is a more effective immunogen than TbpB.

A highly conserved minor outer membrane protein has been discovered via a novel technique using combinations of outer membrane protein preparations from different meningococcal strains to immunize mice (Martin D, et al. 1997. Highly Conserved Neisseria meningitidis Surface Protein Confers Protection against Experimental Infection. J Exp Med 185 (7): 1173-83). The B cells from the mice were used to produce hybridomas which were then screened for cross-reactivity against multiple strains of meningococci. One highly cross-reactive monoclonal antibody was found to bind to a 22 kDa outer membrane protein that was designated NspA. Immunization with recombinant NspA protein was shown to induce a cross-reactive bactericidal response in mice against strains from serogroups A-C. Vaccination also protects mice against lethal meningococcal infection (Martin D, et al. 1997. Highly Conserved Neisseria meningitidis Surface Protein Confers Protection against Experimental Infection. J Exp Med 185 (7): 1173-83). Comparison of NspA sequences among genetically divergent meningococcal strains demonstrates that the protein is highly conserved (97% homology) (Cadieux N, et al. 1999. Bactericidal and Cross-Protective Activities of a Monoclonal Antibody Directed against Neisseria meningitidis NspA Outer Membrane Protein. Infect Immun. 67 (9): 4955-9).

The presence of NspA was detected by ELISA on 99.2% of tested strains from serogroups A-C using anti-NspA monoclonal antibodies (Martin D, et al. 1997. Highly Conserved Neisseria meningitidis Surface Protein Confers Protection against Experimental Infection. J Exp Med 185 (7): 1173-83). These monoclonal antibodies have been shown to be bactericidal against numerous strains of meningococci and are able to reduce meningococcal bacteraemia in a mouse model (Cadieux N, et al. 1999. Bactericidal and Cross-Protective Activities of a Monoclonal Antibody Directed against Neisseria meningitidis NspA Outer Membrane Protein. Infect Immun 67 (9): 4955-9). Although this data appears to suggest that NspA is a promising vaccine candidate that is able to protect across serogroup boundaries, polyclonal anti-recombinant NspA serum from mice does not bind to the surface of around 35% of pathogenic serogroup B meningococcal strains despite the presence of the *nspA* gene in these organisms (Moe GR et al. 1999. Differences in Surface Expression of NspA among Neisseria meningitidis Group B Strains. Infect Immun. 67 (11): 5664-75).

*The N. meningitidis* genome sequence and its impact in vaccine design

The genome sequences of MC58 (a serogroup B meningococcus) (Tettelin H, et al. 2000. complete Genome Sequence of Neisseria meningitidis Serogroup B Strain MC58. Science 287 (5459): 1809-15172) y and of Z2491 (a serogroup A strain) (Parkhill J, et al. 2000. complete DNA sequence of a serogroup A strain of Neisseria meningitidis Z2491. Nature 404 (6777):502-6173) were elucidated and published during 2000. The availability of the annotated gene sequences should have a dramatic influence on meningococcal vaccine research. While the MC58 genome sequencing was in progress, Pizza et al. began identifying the open reading frames that were predicted to encode either membrane bound, surface exposed or exported proteins. They identified 570 such ORFs, amplified them via the polymerase chain reaction and cloned them into *Escherichia coli* to allow expression of the encoded proteins as either His-tagged or glutathione S-transferase fusion proteins (Pizza M, et al. 2000. Identification of Vaccine Candidates Against Serogroup B Meningococcus by Whole-Genome Sequencing. Science 287 (5459): 1816-20). The 61% (350) of the selected ORFs were successfully expressed, those which failed to express were often those containing more than one hydrophobic trans-membrane domain (possibly excluding a number of outer membrane bound proteins). The recombinant proteins were purified and used to vaccinate mice. The immune sera were then assessed for surface binding to multiple meningococcal strains by enzyme linked immunosorbent assay (ELISA) and flow cytometry and for bactericidal activity against two strains using the serum bactericidal assay. Finally seven proteins were selected for further study on the basis of a positive response in all three assays. Trial vaccine formulations using a number of these proteins in combination with adjuvants have been shown to induce significant bactericidal tires against the homologous meningococcal strain (MC58) in mice, but none of the proteins induced SBA litres as high as an MC58 outer membrane vesicle vaccine (Giuliani MM, et al. 2000. Proceedings 12th IPNC. p. 22). On the other hand, there is some evidence that combinations of these proteins may exhibit higher immunogenicity in mice than single proteins (Santini L. et al. 2000. Proceedings 12th IPNC. p. 25). The numerous open reading frames which were excluded during this work, perhaps through failure of protein expression or modification of their immunological properties, may also have vaccine potential and require further investigation.

Vaccine components may be selected more effectively once an understanding of the contribution of individual antigens to the pathogenesis of *N. meningitidis* has been gained. The antigens themselves may make effective vaccine candidates or, alternatively, the attenuated mutants could be considered as vaccine constituents.

An important problem of meningococcal disease prevention and//or therapy is that no available vaccine to date confers a universal protection due to the heterogeneity of antigens used as vaccines so far.

### Description of the invention

This invention contributes to solve the problem mentioned before, by supplying pharmaceutical formulations containing a protein which sequence is highly conserved, even in different pathogenic bacterial genus. The technical objective that this invention pursues is the development of formulations with the ability to increase the systemic and mucosal host immune response against different new pathogens or a wider spectrum of existing ones. In the work object of the present invention it is reported, for the first time, the use of the NMA0939 protein as a component of a vaccine formulation with therapeutic or preventive character against the meningococcal disease or any infection caused by a member of the *Neisseria* genus. The novel character of this invention consists in the use, previously unreported, of the NMA0939 protein in formulations with new properties, able to induce a systemic and mucosal immune response of broad-spectrum protection, due to the conserved character of this protein in different isolates of *Neisseria meningitidis* and *Neisseria gonorrhoeae*. In another realization of this invention, pharmaceutical compositions might contain one or several antigens being of synthetic, recombinant or natural origin. In another realization of this invention, combined pharmaceutical compositions could contain polysaccharide antigens, including bacterial polysaccharides, and more specifically, N. meningitidis polysaccharides. Formulations of the present invention can contain conjugated protein-polysaccharides, being the polysaccharide of bacterial origin.

In one preferred realization of the present invention, pharmaceutical formulations containing NMA0939 also contain antigens of peptide nature, with the porpoise of expanding the protection spectrum induced by vaccines derived from said compositions.

Pharmaceutical formulations described herein are administered by parenteral or mucosal routes, including oral route.

In another preferred realization of the present invention, NMA0939 protein can be employed as adjuvant, or carrier of peptides, polysaccharides, or any other antigen with lesser immunogenicity, aiming to boost the immunogenicity of said elements. Example 11 shows that NMA0939 protein is capable to enhance the antibody levels against a viral-derived peptide when said peptide is conjugated to NMA0939. It is also comprised within the scope of the present invention to cover the use of protective determinants for a protein antigen given that they are inserted into the NMA0939 amino acid sequence, aiming to induce an enhanced immune response against such determinants, thus being part of new hybrid proteins present in a pharmaceutical composition.

In another preferred realization of the present invention, pharmaceutical compositions comprised in the present invention might contain NMA0939 protein fragments, capable to induce a protective response against the meningococcus or any other bacteria of the Neisseria genus. In a particular realization of the present invention, pharmaceutical compositions contain NMA0939 mimotopes or NMA0939 mimetic peptides generated by synthesis or recombinant DNA technology. The term "mimotope" describes herein any peptide being able to induce antibodies, and that they are combined with NMA0939 protein while being able to induce a protective immune response against Neisseria.

It is also a part of present invention the detection of meningococcal disease through the use of pharmaceutical components containing NMA0939, or the NMA0939 coding gene, along or in combination with other components.

### Brief description of drawings

**Figure 1****.** Cloning vector pM238 employed in the cloning and expression of protein NMA0939.
**Figure 2****.** Final construction of nucleotide sequence of the gene *NMA0939* in pM238 vector.
**Figure 3**. SDS-PAGE analysis of fractions obtained from cellular disruption: lane 1, whole cells; lane 2, cellular pellet alter disruption; lane 3, supernatant after disruption; PM: Molecular Weight Markers.
**Figure 4****.** SDS-PAGE analysis of the different fractions of purification process solubilization process of recombinant protein NMA0939 starting from the disruption pellet: Lane 1, pellet after solubilization with 2M urea in carbonate-bicarbonate buffer; lane 2, supernatant of solubilization; lane 3, unbound fraction coming out from purification matrix; lane 3, low molecular weight contaminant released from matrix at a different elution peak; lane 5, purified protein. PM: molecular weight marker.
**Figure 5****.** Antibody levels (IgG) against recombinant protein NMA0939, obtained after mice immunization with the same antigen adjuvated with Freund's adjuvant (Freund), aluminum hydroxide (Alum) or *N. meningitidis* C polysaccharide by intra-peritoneal route. ELISA results are represented, as the inverse of the highest dilution that duplicates the value of pre-immune sera.
**Figure 6****.** Recognition of antigenic determinants present in *N. meningitidis,* strain CU385, OMVs using murine sera obtained after mice immunization with the same antigen adjuvated with Freund's adjuvant (Freund), aluminum hydroxide (Alum) or *N. meningitidis* C polysaccharide by intra-peritoneal route. Results are represented, as the inverse of the highest dilution that duplicates the value of pre-immune sera.
**Figure 7****.** Results of homology searches between NMA0939 protein ("query") and annotated sequences in genomes from different serogroups of *N. meningitidis* ("Sbjct") using the BLAST program.
**Figure 8****.** Recognition of NMA0939 protein in five different strains of *N. meningitidis,* by sera elicited against the recombinant antigen adjuvated with *N. meningitidis* C polysaccharide by intra-peritoneal route. Sera elicited with other adjuvants had a similar profile. Results are expressed as the inverse of the highest dilution that duplicates the value of pre-immune sera.

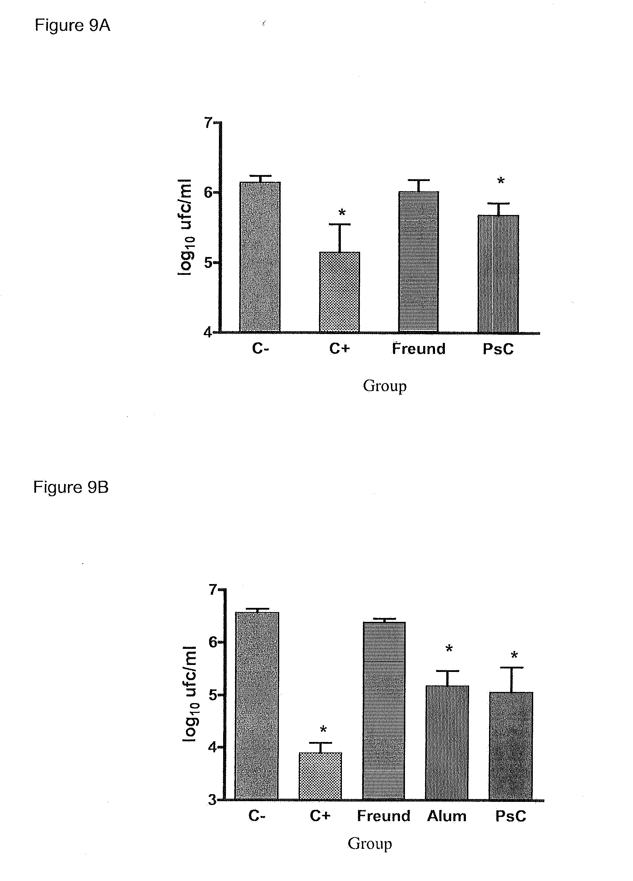

**Figure 9****.** Meningococcal infection passive protection experiments in the infant rat model using sera elicited against the recombinant antigen adjuvated with Freund's adjuvant (Freund), aluminum hydroxide (Alum) or *N. meningitidis* C polysaccharide (PsC). **A:** Infection with CU385 strain, and **B:** Infection with strain 233 (C: 2a: P1.5). **C-:** pool of untreated animals, C+: hyper immune mice sera against outer memebrane vesicles of Esther CU385 or 233 strains depending on the experiment. The symbol * represents a significant statistical difference in respect to the negative control (C-), Regarding to the levels of bacteraemia, these are expressed as colony forming units per mL (cfu/ml).
**Figure 10****:** Recognition of protein NMA0939 and a panel of un-related antigens by generated mAbs (mAbs H10/67, 3H3/24 y 7D6/18). P1, Class 1 protein *Neisseria meningitidis* strain B:4:P1.15; P64k, E3 subunit of pyruvate dehydrogenase from *Neisseria meningitidis*; T.T, tetanus toxoid; HBsAg, Hepatitis B surface Antigen. These results are shown as absorbance values (492 nm) in an ELISA-type assay.
**Figure 11****.** Recognition of NMA0939 protein by human convalescent sera from survivors of meningococcal disease. As negative control healthy donor sera were employed. Results are shown as the absorbance (492nm) in an ELISA type assay.
**Figure 12****.** JY1 anti-peptide titers from the sera of animals immunized with either free peptide (JY1), recombinant protein (NMA0939) or the conjugate JY1- NMA0939.
**Figure 13****.** Antibody Levels (IgA) against recombinant NMA0939 protein in pulmonary wash samples from intranasal immunized mice with a recombinant NMA0939 N. meningitidis C polysaccharide (NMA0939+PsC) or with the protein incorporated into liposomes (NMA0939_Lip).

### Examples for realization

### Example 1. Detection of NMA0939 protein in serogroup B Neisseria meningitidis outer membrane vesicles preparations

With the aim of studying proteins that are present in serogroup B Neisseria meningitidis (strain B:4:P1.19,15) outer membrane vesicles, a bi-dimensional electrophoresis was carried out according to a method described elsewhere (Görg A, et al. 1985. Electrophoresis 6:599-604). Subsequently an enzymatic digestion was made upon the gel extracted proteins using trypsin (Promega, Madison, WI, U.S.). Peptides generated after digestion were extracted into solution by using micro columns (ZipTips, Millipore, MA, U.S.). For mass spectrometry analysis peptides were eluted from micro columns with acetonitrile 60%, formic acid 1% followed by an immediate application into nanotips (Protana, Denmark).

Measurements were carried out in a hybrid mass spectrometer with quadrupole and time of flight (QTof-2^{™}, Manchester, United Kingdom), fitted with an ionization source (nanoESI). Mass spectrometry data were acquired in a w/z range of 400-2000 in 0.98 seconds and using 0.02 seconds between scannings. Data acquisition and data processing were carried out using the MassLynx program (version 3.5, Micromass).

Protein identification based on mass spectrum data was carried out using the ProFound program (Zhang W and Chait BT. 2000. ProFound: an expert system for protein identification using mass spectrometric peptide mapping information. Anal Chem 72:2482-2489. http://prowl.rockefeller.edu/cgi-bin/ProFound). The search was subscribed to the genes and derived protein sequences contained in the SwissProt database (http://www.ebi.ac.uk/swissprot/) and NCBI (http://www.ncbi.nlm.nih.gov/), considering the oxidation of methionines, deamidation and carboxyamidomethylation of cysteines as possible modifications to be encountered.

Identification of proteins based on the mass spectra was carried out with the MASCOT program (Perkins DN, et al. 1999. Probability-based protein identification by searching sequence databases using mass spectrometry data. Electrophoresis 20:3551-3567. http://www.matrixscience.com/). Search parameters included cysteine modifications as well as oxidations and deamidations.

Starting from the analysis of results obtained from the identification of proteins present in preparations of outer membrane vesicles a group of peptides was selected for further sequencing.

From the sequence data obtained from one peptide the presence of NMA0939 protein homologue in serogroup B was confirmed, not being predicted or identified in the previous published genomes. This serogroup B protein homologue to NMA0939 it is called NMA0939 throughout the entire document.

### Example 2. Homology-based analysis of NMA0939 protein with gene products reported in databases.

For the identification of the NMA0939 protein, a sequence homology search was done in the NCBI data base employing the BLAST program (Altschul SF, et al. 1990. Basic local alignment search tool. J Mol Biol 215:403-410, http://www.ncbi.nlm,nih.gov/BLAST/). This procedure allowed us to identify the *N. gonorrhoeae* homologue and the serogroup A *N. meningitidis* gene, not being this case the same for MC58 serogroup B strain. However, the performed search using the NMA0939 DNA coding sequence, nma0939 gene, rendered the identification of the presence of such gene in MC58 reported genome, thus indicating an error in the annotation of this genome. The serogroup B gene detected is also homologues to *N. gonorrhoeae* (NGO0306) nma0939-homologue. In serogroup B, the reported gene detected in this work is located in a DNA region between positions 761673 and 762295, downstream *nmb0729* gene and upstream *nmb0730* gene. Thus, this is so far the first report of the existence of a NMA0939 protein homologue in serogroup B meningococci. In different microorganisms homologue proteins were not successfully detected thus indicating that NMA0939 is a Neisseria-specific antigen.

### Example 3. Cloning and expression of the nma0939 gene, codifying for NMA0939 protein from N. meningitidis in Escherichia coli.

In order to clone and express the NMA0939 gene, the pM-100 cloning vector was employed. This vector allows the cloning to be carried out using different restriction enzymes and the generation of high expression levels of heterologous proteins in the form of inclusion bodies in *E. coli.*

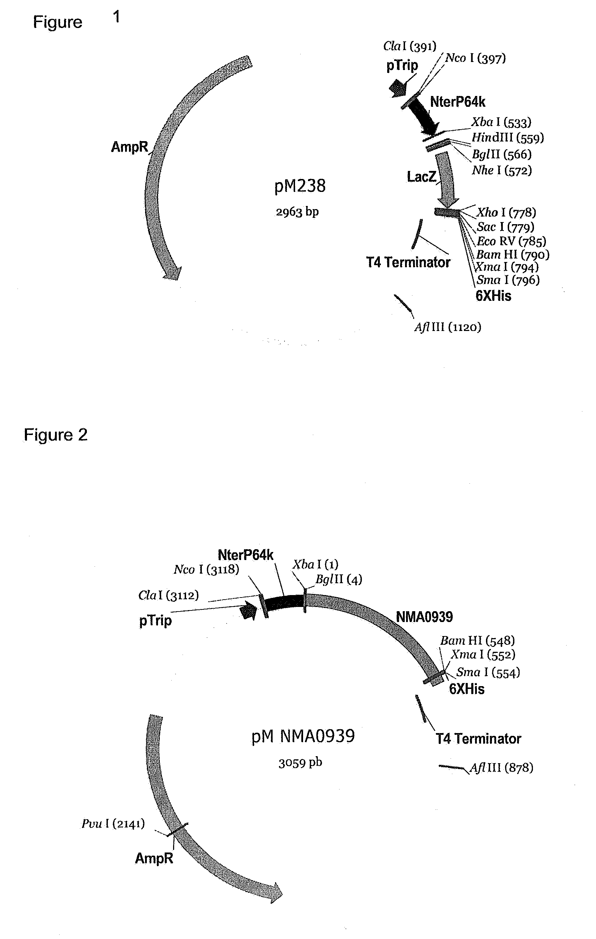

The pM-100 vector (Figure 1) have the following elements: tryptophan promoter, gene segment codifying for the 47 amino acid stabilizing sequence from Nt-fragment of P64 kDa from *N. meningitidis* strain B:4:P1.19,15, sequence of bacteriophage T4 transcriptional terminator, and the sequence of the gene that confers resistance to Ampicillin as selection marker. This cloning vector also allows recombinant selection by the means of a blue/white color staining of transformed colonies, due to the presence of the beta-galatosidase lacZ alpha subunit.

From NMA0939 coding gene nucleotide sequence (Example 1) a oligonucleotide primer pair (704467U and 704467L) was designed for amplification of said gene segment, avoiding the signal peptide coding region, from strain CU385 (B:4:P1.19,15) genomic DNA. 
Xba-I Bgl-II
704467U: 5`GTGGTATCTAGATCTGCCAGCCAGAAACTC `3
(SEQ ID No. 1)
 
704467L: 5` CAACCCGGGATCCTTCCTTGTCCAAATC 3`
BamH-I
(SEQ ID No. 2)
 For the prediction of signal peptide the SignalP World Wide Web server (http://www.cbs.dtu.dk/services/SignaIP-2.0) was employed.

After PCR amplification of the NMA0939-coding gene (Saiki R K, et al. (1988). S Primer-directed enzymatic amplification of DNA with a thermostable DNA polymerase. Science 239: 487-491) employing primers 7740 and 7741, the PCR product was digested using Bgl-II and Bam-HI restriction enzymes, and cloned into vector previously digested pM238 cloning vector. The final construction is showed in Figure 2, and the NMA0939 protein is expressed as a fusion protein to the Nt-segment of P64 kDa protein. Sequencing of the cloned gene nma0939 was carried out using ALFexpress II automatic sequencer (Termo Sequenase^{™} Cy^{™} 5 Dye Terminador Kit, Amersham Biosciences) and oligonucleotides 1573 (Seq. ID. No. 8) and 6795 (Seq. ID. No. 9) that bind the sequence of the P64 stabilizer and T4 transcriptional terminator, respectively. The plasmid generated herein was designated pM-NMA0939 for later use.

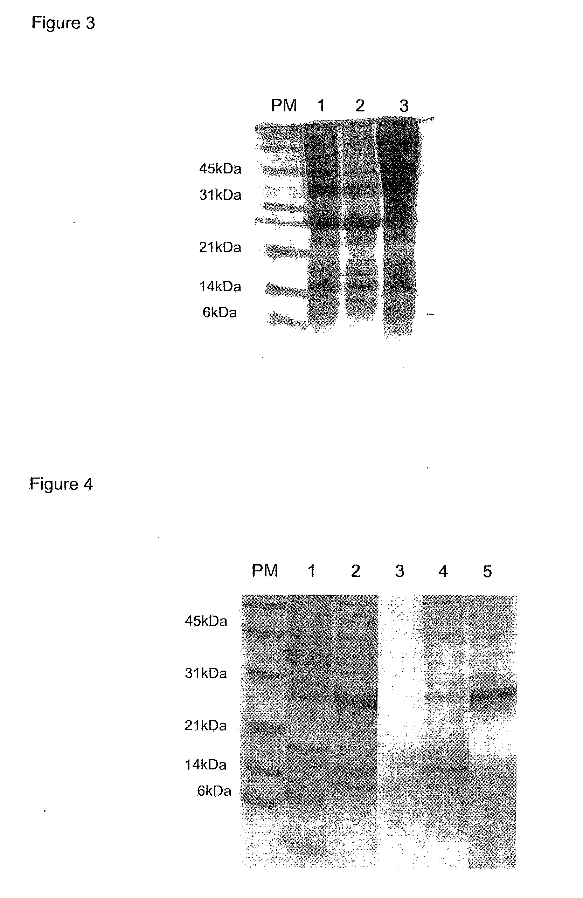

For the expression of the NMA0939 gene the GC366 *E. coli* strain was transformed by the chemical method with the pM-NMA0939 plasmid (Figure 2). The expression experiment was carried out in minimal media (M9) (Miller JH. 1972. Experiments in Molecular Genetics, Cold Spring Harbor Laboratory Press, NEW York, USA) supplemented with 1% glycerol, 1% casein hydrolyzed, 0.1 mM CaCl₂, 1mM MgSO₄ and 50 ug/mL ampicillin. Bacterial cultures were incubated 12 hours at 37 °C and 250 rpm. Grown cultures were centrifuged and ultrasonic disruption of the cellular pellet was performed (IKA LABORTECHNIK). Fractions from pellet and supernatant were analyzed by SDS-PAGE (Laemmli UK. 1970. Cleavage of structural proteins during the assembly of the head of bacteriophage T4. Nature 277:680) plus stain with Coomassie Brilliant Blue R-250. The percent of expression was carried out by gel densitometry (LKB Bromma 2202 Ultrascan laser densitometer; Amersham Pharmacia Biotech, United Kingdom). The NMA0939 protein was obtained from the pellet fraction, being about the 20% of total protein content of this fraction (Figure 3). Samples of cellular pellet were solubilized in carbonate-bicarbonate buffer (sodium carbonate 0.1M, sodium hydrogen-carbonate 0.1M) containing urea at different molar concentrations (2M, 4M, 6M and 8M). When the previous buffer with 2M urea was employed, the NMA0939 protein was solubilized. Supernatant after solubilization went through a metal affinity chromatography in order to achieve the purification of the protein of interest. As a result a sample with a purity of 80% was obtained as it shown in Figure 4. A last step of dialysis was performed before its evaluation in lab animals.

### Example 4. Evaluation of the immune response induced after immunization with NMA0939 protein by intra-peritoneal route.

To evaluate the immunogenicity of the protein NMA0939, an immunization experiment was designed and conducted in mice, where the NMA0939 protein was administered adjuvated with Aluminum Hydroxide, Freund's Adjuvant, or *N. meningitidis* C polysaccharide.

With these preparations, female Balb/C mice (8-10 weeks-old) were immunized, once divided in 4 groups of 8 mice, each. Three immunizations were applied by intra-peritoneal route, with 7 days-interval in between. A control group was employed and consequently immunized with PBS, however, like the other groups in the experiment, a booster dose with the protein adjuvated in Aluminum hydroxide was given at day 45. In Table 1 is described the composition of the groups:

**Table 1: Balb/C mice groups employed in the immunization experiment**

| Group | Immunogen |
|---|---|
| 1 | 20µg NMA0939+ Freund's Adjuvant |
| 2 | 20µg NMA0939 + Aluminum Hydroxide |
| 3 | 20µg NMA0939+ C polysaccharide |
| 4 | PBS |

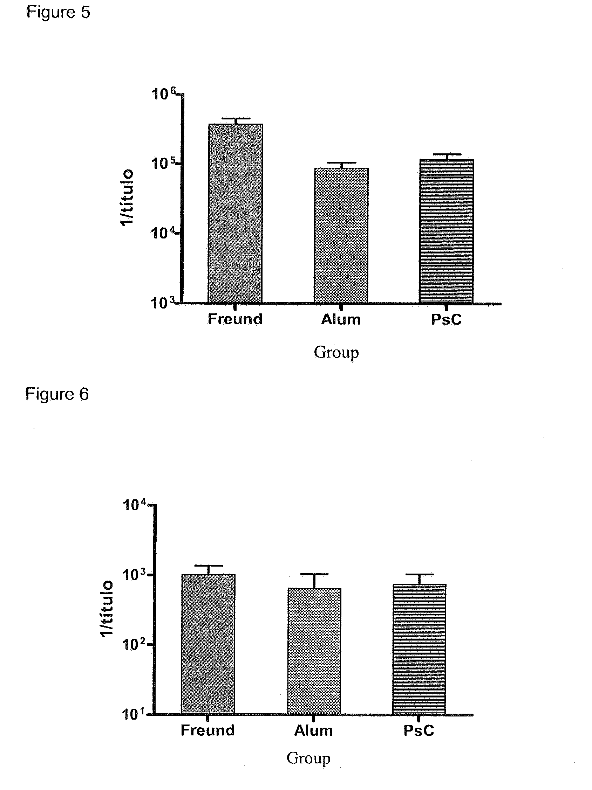

Antibody titers (IgG) against the recombinant protein and the homologous protein present in the bacterium were determined by an ELISA, in serum samples taken after the booster dose. In Figure 5, the antibody titers against the recombinant protein of individual animals are shown. Specific antibody levels were detected right after the second inoculation (data not shown), although they were higher after the last inoculation. Moreover, the immunoidentification by *Western blotting* was done, where the respective protein band was recognized (data not shown). The groups immunized by intra-peritoneal route had titers significantly higher than those elicited by intra-nasal route.

The sera obtained after the immunization with the recombinant protein recognized the natural protein present in a preparation of outer membrane protein (OMP) of strain CU385. These results are represented in Figure 6.

For the statistical analysis of the results, a log transformation of antibody titers was performed in order to have a normal distribution of variance and its homogeneity. In general, statistical signification of recorded differences among the group means was performed through a simple ANOVA followed by a multiple rank test of Neuman Keuls.

### Example 5. Characterization of the sequence of the gene codifying for protein NMA0939 in different strains of N. meningitidis and Neisseria gonorrhoeae.

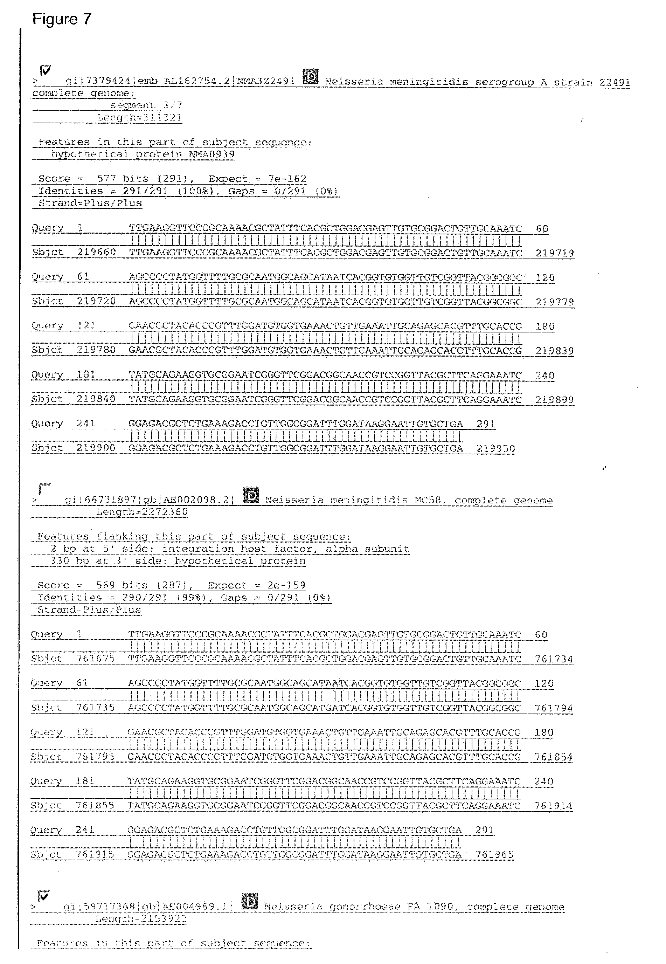

To analyze the conservation of the sequence of the gene codifying for the NMA0939 protein in the pathogenic species of the Neisseria genus a similarity search with the genomes of *Neisseria meningitidis* (serogroups A, B and C) and *Neisseria gonorrhoeae,* annotated in the NCBI data base, was done (NC 003116.1, NC 003112.1, NC 003221, NC 002946) employing the BLAST program (Altschul SF, et al. 1990. Basic local alignment search tool. J Mol Biol 215:403-410. http://www.ncbi.nlm.nih.gov/BLAST/). Figure 7 shows the results of the sequence comparison for those sequences that produce a significant aligment in each of the analyzed genomes. Those sequences have 99% identity in serogroup B, 100% identity in serogroup A and a 96%identity with *Neisseria gonorrhoeae*, with the sequence obtained for the gene that codifies for the NMA0939 protein (Seq. ID. No. 3). In addition, the sequence of the referred gene was determined for 3 Cuban isolates (Seq. ID. No. 5-7), which belong to serogroup B (B:4-P1.19,15) and a sequence alignment was done by using the ClustalX program (http://www.ebi.ac.uk/clustalw/). The results of the alignment show that there is a great conservation in the nucleotide sequence of the gene NMA0939 among the analyzed strains and in general in the *Neisseria* gender.

The use of the protein NMA0939 as a vaccine candidate, taking into account the high degree of similarity existing among the sequences previously mentioned, would allow the generation of an effective immune response, with a broad-spectrum protection (due to the cross reactivity) against the meningococcal disease.

### Example 6. Characterization of the immune response with broad-spectrum action induced by the immunization of Balb/C mice with the protein NMA0939.

To evaluate if the immunization with protein NMA0939 induced a response broadly cross-reactive with other strains of Neisseria, an ELISA was done. The polystyrene plates were coated with whole cells of 7 strains of Neisseria, which belong to different serotypes and serosubtypes. The plates were incubated with pooled sera obtained against the protein NMA0939, by two routes of immunization, as described in Example 4.

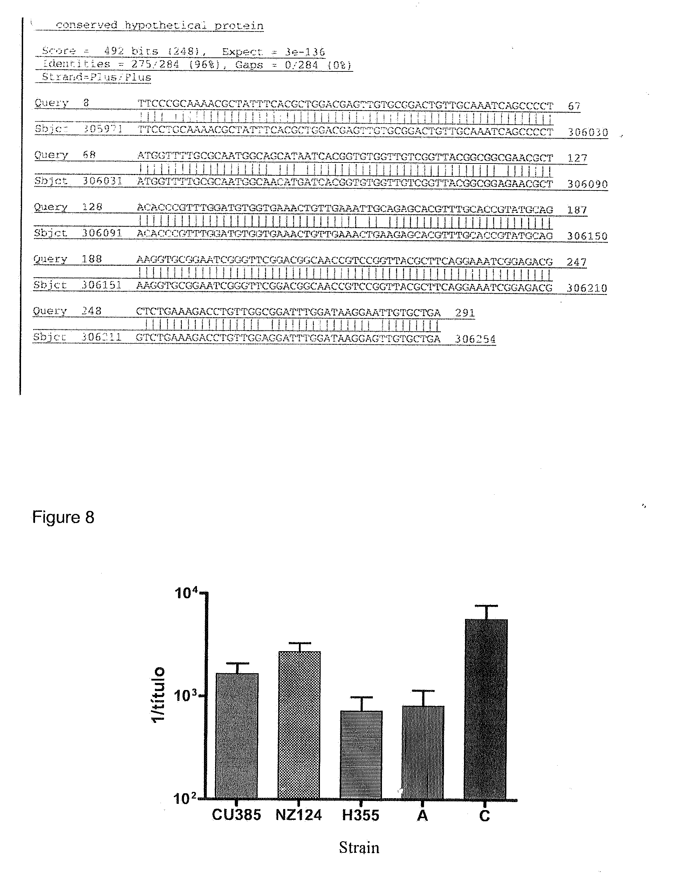

Figure 8 shows the recognition of antigens present in strains from serogroups A, B, and C from N. meningitides by the sera elicited after the immunization with the recombinant NMA0939 protein adjuvated with C polysaccharide. As it is observed, the immune sera recognized the protein present in different strains, with levels similar to the one found in the strain CU385. The rest of the sera had a comparable behavior in this assay.

### Example 7. Protection induced by the murine sera specific for protein NMA0939, against homologous and heterologous strains, in the infant rat model.

To determine the functional activity of the anti-sera obtained, a protection assay was conducted in the infant rat model for meningococcal infection. Twenty four rats (5-6 days old) were divided in groups of 6 rats each.

It was determined if the sera administered by intra-peritoneal route protected the rats from the infection caused by bacteria (strain CU385), inoculated by the same route one hour later. The sera of each group were pooled and diluted 1/10 (in sterile PBS) before they were inoculated in infant rats. Four hours later, the animals were sampled and viable bacteria in their blood were counted.

To interpret results a single tail student *t* test comparing each group with the negative control group (pool from un-treated animals). As can observed in Figure 9A the group receiving sera from mice immunized with NMA0939 adjuvated with C polysaccharide did show statistically significant differences in comparison with negative control group, thus the antibody titers were protective in the infant rat model. Antibody response generated after immunization with the protein adjuvated with Aluminum Hydroxide was also protective in this animal model (data not shown).

A similar assay was done infecting infant rats with strain 233 (C:2a: P1.5) and results are shown in Figure 9B, finding that, like in the former experiment, antibodies elicited with the target NMA0939 protein adjuvated either with Aluminum Hydroxide or C polysaccharide were able to protect rats against meningococcal infection.

A similar assay was done infecting infant rats with strains H44/48 and 120/90, isolated from Cuban patients, which serological classification is homologous to the strain B385. Moreover, challenge experiments were conducted with strain H44/76 (B:15:P1.7,16) del serogrupo B. In all cases, the antisera containing antibodies elicited with the target NMA0939 protein adjuvated either with Aluminum Hydroxide or C polysaccharide were able to protect rats against meningococcal infection.

### Example 8

### Generation of monoclonal antibodies against protein NMA0939 able of mediating the bactericidal activity against Neisseria meningitidis

To generate monoclonal antibodies (mAbs) specific against protein NMA0939, and study the functional ability of mediating bactericidal activity against homologous and heterologous strain of *N. meningitidis*, an immunization schedule was conducted with a preparation of protein NMA0939 with 80% purity (Example 3). The immunization was done in Balb/C (H-2^{d} , female, 5-6 weeks old) and 4 doses were applied as follows: On days 0, 15 and 30 of the immunization routine, 10 µg of antigen NMA0939 per mouse (total volume 100 µl), were administered by subcutaneous route, emulsified with Freund's Adjuvant; on day 50, 10 µg of antigen per mouse in Phosphate Buffered Saline (140 mM NaCl, 270 mM KCI, 1.5 mM KH₂PO₄, 6.5 mM Na₂HPO₄ x 2H₂O, pH 7.2) were administered by intra-peritoneal route. Blood extractions were done on days 0 and 45.

Splenocytes from the animal with the highest titer, measured by an indirect ELISA using protein NMA0939 as the coating antigen (Example 3), were fused with X63 Ag8 653 mouse myeloma cells. The resulting hybridomas were isolated and screened according to standard procedures (Gavilondo JV. 1995. Anticuerpos Monoclonales: Teoría y Práctica, Elfos Scientiae, La Habana, Cuba).

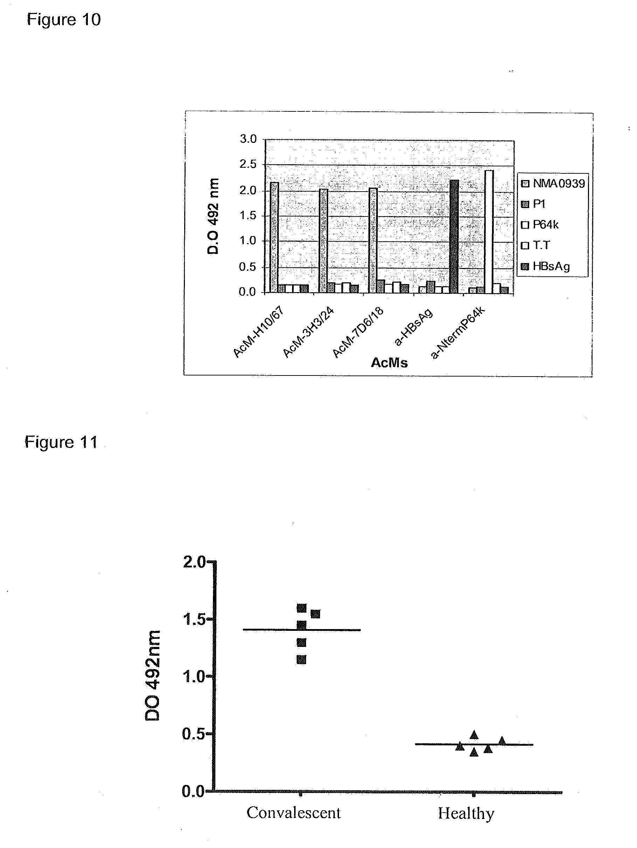

The reactivity of the antibodies secreted by the hybridomas directed to protein NMA0939, as well as their cross-reactivity non-related antigens, was tested by an indirect ELISA employing 5 µg/ml of each antigen, and the same concentration of each mAbs to be assayed. Figure 10 shows the results obtained in this experiment, all together 2 positive clones were obtained (mAbs H10/67, 3H3/24 and 7D6/18) which specifically recognized protein NMA0939, and do not react neither with the amino acid sequence corresponding to the N-terminal of P64k, nor with the rest of the non-related antigens assayed.

To determine the ability of the mAbs generated against protein NMA0939 to mediate a bactericidal response against homologous and heterologous strains of *Neisseria meningitidis* a bactericidal test was performed. The bactericidal antibody titer was expressed as the reciprocal of the highest dilution of the antibodies tested that was able of killing 50% or more bacteria; two of the mAbs (3H3/24 y 7D6/18) achieved bactericidal titers higher than 1:128 against the homologous strain B:4:P1.19,15 and one (H10/67) a titer higher than 1:80. They also showed titers higher than 1:64 against the heterologous strains B:15:P1.7,16 and C:2a:P1.5 respectively.

### Example 9 Characterization of the target regions of the murine immune response against protein NMA0939

In order to identify the regions in the protein, which are more frequently recognized by the murine anti-sera generated against the recombinant antigen a SPOTScan assay was done. A set of overlapping peptides that span the sequence of the protein was synthesized on a cellulose membrane, which was incubated with pooled sera diluted 1:100. The antigen-antibody reaction was detected by the incubation with a conjugate anti-murine immunoglobulin G- alkaline phosphatase, followed by the addition of a solution that contained the substrate Bromo-chloro-indolyl-phosphate.

Several antigenic regions common within the protein were observed, no matter the preparation that was employed for the immunization. However, in the groups immunized with the protein adjuvated with Freund's Adjuvant there was a much broader pattern of recognition.

### Example 10. Recognition of the NMA0939 protein by human sera.

A collection of human sera, coming from convalescent individuals was employed in this study, which was performed by ELISA. The plates were coated with protein NMA0939, obtained by preparative electrophoresis (5 µg/ml). Alter blocking the plates with 3% skim milk powder in PBS containing Tween-20, the sera were diluted (1:50) in the same solution and were incubated in the plates. The immunoassay continued as it has been widely reported. Healthy donor sera were employed as negative controls. In addition, pooled sera from individuals vaccinated with a recombinant vaccine against Hepatitis B was used a non-related control (data not shown).

Figure 11 shows the results obtained with 5 convalescent's sera in this assay. It can be seen that the human sera recognized the protein, which indicates that it is expressed during the meningococcal infection and it is immunogenic.

### Example 11. Protein NMA0939 as a carrier for a peptide.

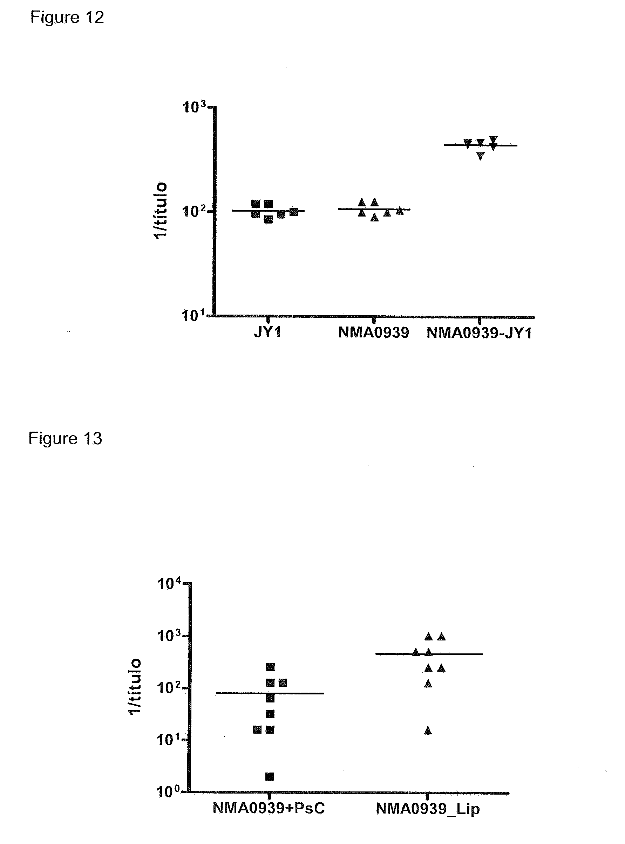

To demonstrate the carrier capacity of the recombinant protein NMA0939, it was conjugated to a 15 mer synthetic peptide, derived from the V3 region of protein gp120 from HIV-1, isolate JY1. The conjugation was done by the glutaraldehyde method. Free JY1 peptide, the recombinant protein NMA0939 and the conjugate JY1- NMA0939, were administered to adult mice in a 3-dose schedule, where the immunogens were emulsified with Freund's Adjuvant. Two weeks after the third dose, serum samples were obtained from the immunized animals, and the samples were analyzed by ELISA to determine the anti-peptide antibody titers. To do that, the plates were coated with free peptide (20µg/ml) and the immunoassay continued as it has been previously described. The results of the experiment (Figure 12) show the carrier capacity of protein NMA0939, able of significantly potentiate the antibody response against peptide JY1, after their conjugation.

### Example 12. Evaluation of the immune response induced after immunization with NMA0939 protein by mucosal route.

To evaluate the immunogenicity of the protein NMA0939, an immunization experiment was designed and conducted in mice, where the NMA0939 protein was administered encapsulated into liposomes or adjuvated with *N. meningitidis* C polysaccharide. Liposomes were obtained by dehydration-rehydration as previously described (Carmenate T, et al. (2001). Recombinant Opc protein from Neisseria meningitidis reconstituted into liposomes elicits opsonic antibodies following immunization. Biotechnol. Appl. Biochem. 34: 63-69). With these two preparations, female Balb/C mice (8-10 weeks-old) were immunized. Three doses of 50 µg via intranasal route were applied, with 15 days-interval in between. In order to analyze the immune response at mucosal level, IgA antibody levels were measured in pulmonary washes of immunized animals. In Figure 13 the detected IgA antibody levels are shown for the two groups evaluated.

## Claims

1. Pharmaceutical formulation **characterized by** containing the protein NMA0939 identified as Seq. ID. No 4.

2. Pharmaceutical formulation according to claim 1 **characterized by** being a vaccine able to generate in the recipient organism a protective response against infections caused by bacteria from the *Neisseria* genus.

3. Pharmaceutical formulation according to claim 1, **characterized by** being a vaccine able to generate in the recipient organism a protective response against infections caused by *Neisseria meningitidis or Neisseria gonorrhoeae*.

4. Pharmaceutical formulation according to claim 1, **characterized by** containing in addition one or several antigens of different nature obtained by recombinant, synthetic, or natural means.

5. Pharmaceutical formulation according to claim 4, **characterized by** containing polysaccharide antigens, including bacterial polysaccharides.

6. Pharmaceutical formulation according to claim 5, **characterized by** containing *Neisseria meningitidis* capsular polysaccharides.

7. Pharmaceutical formulation according to claim 4, **characterized by** containing protein-polysaccharide conjugates in which the polysaccharide component is a bacterial polysaccharide.

8. Pharmaceutical formulation according to claim 4, **characterized by** containing peptide antigens.

9. Pharmaceutical formulation according to claim 1, to administered by parenteral or mucosal routes.

10. Pharmaceutical formulation according to claim 1, **characterized by** the use of NMA0939 protein as an adjuvant or carrier for antigens of different nature.

11. Pharmaceutical formulation **characterized by** containing peptide fragments, or mimetic peptides of NMA0939 protein antigen.

12. Pharmaceutical formulation **characterized by** containing NMA0939 protein identified as Seq. ID. No 4, to be used in the detection of meningococcal disease in humans, either alone or in combination with other components.

13. Pharmaceutical formulation **characterized by** containing NMA0939 coding gene, identified as Seq. ID. No 3, to be used in the detection of meningococcal disease in humans, either alone or in combination with other components.


SEQUENCE LIST

<110> Center for Genetic Engineering and Biotechnology

<120> Pharmaceutical formulations containing NMA0939 protein

<130> P85109NO00

<150> PCT/CU2006/000019
<151> 2006-12-28

<150> CU 2005-0279
<151> 2005-12-29

<160> 9

<170> PatentIn Ver. 2.1

<210> 1
<211> 30
<212> ADN
<213> Artificial Sequence

<220>
<223> Artificial Sequence Description: Oligo
      704467U

<400> 1
gtggtatcta gatctgccag ccagaaactc                                   30


<210> 2
<211> 28
<212> ADN
<213> Artificial Sequence

<220>
<223> Artificial Sequence Description:Oligo
      704467L

<400> 2
caacccggga tccttccttg tccaaatc                                     28


<210> 3
<211> 348
<212> ADN
<213> Neisseria meningitidis (group B)

<400> 3
atgccagcca gaaactcaaa agcatggtgg aacactacta tgacaaacaa cgttgatttg 60
aaggttcccg caaaacgcta tttcacgctg gacgagttgt gcggactgtt gcaaatcagc 120
ccctatggtt ttgcgcaatg gcagcatgat tatggtgtgg ttgtcggtta cggcggcgaa 180
cgctacaccc gtttggatgt ggtgaaactg ttgaaattgc agagcacgtt tgcaccgtat 240
gcagaaggtg cggagtcggg ttcggacggc aaccgtccgg ttacgcttca ggaaatcgga 300
gacggtctga aagagctgtt ggcggatttg gataaggaat tgtgctga               348


<210> 4
<211> 115
<212> PRT
<213> Neisseria meningitidis (group B)

<400> 4
Met Pro Ala Arg Asn Ser Lys Ala Trp Trp Asn Thr Thr Met Thr Asn
  1               5                  10                  15

Asn Val Asp Leu Lys Val Pro Ala Lys Arg Tyr Phe Thr Leu Asp Glu
             20                  25                  30

Leu Cys Gly Leu Leu Gln Ile Ser Pro Tyr Gly Phe Ala Gln Trp Gln
        35                  40                  45

His Asp Tyr Gly Val Val Val Gly Tyr Gly Gly Glu Arg Tyr Thr Arg
    50                  55                  60

Leu Asp Val Val Lys Leu Leu Lys Leu Gln Ser Thr Phe Ala Pro Tyr
65                  70                  75                  80

Ala Glu Gly Ala Glu Ser Gly Ser Asp Gly Asn Arg Pro Val Thr Leu
                85                  90                  95

Gln Glu Ile Gly Asp Gly Leu Lys Glu Leu Leu Ala Asp Leu Asp Lys
            100                 105                 110

Glu Leu Cys
        115


<210> 5
<211> 348
<212> ADN
<213> Neisseria meningitidis (group B)

<400> 5
atgccagcca gaaactcaaa agcatggtgg aacactacta tgacaaacaa cgttgatttg 60
aaggttcccg caaaacgcta tttcacgctg gacgagttgt gcggactgtt gcaaatcagc 120
ccctatggtt ttgcgcaatg gcagcatgat tatggtgtgg ttgtcggtta cggcggcgaa 180
cgctacaccc gtttggatgt ggtgaaactg ttgaaattgc agagcacgtt tgcaccgtat 240
gcagaaggtg cggagtcggg ttcggacggc aaccgtccgg ttacgcttca ggaaatcgga 300
gacggtctga aagagctgtt ggcggatttg gataaggaat tgtgctga             348


<210> 6
<211> 348
<212> ADN
<213> Neisseria meningitidis (group B)

<400> 6
atgccagcca gaaactcaaa agcatggtgg aacactacta tgacaaacaa cgttgatttg 60
aaggttcccg caaaacgcta tttcacgctg gacgagttgt gcggactgtt gcaaatcagc 120
ccctatggtt ttgcgcaatg gcagcatgat tatggtgtgg ttgtcggtta cggcggcgaa 180
cgctacaccc gtttggatgt ggtgaaactg ttgaaattgc agagcacgtt tgcaccgtat 240
gcagaaggtg cggagtcggg ttcggacggc aaccgtccgg ttacgcttca ggaaatcgga 300
gacggtctga aagagctgtt ggcggatttg gataaggaat tgtgctga             348


<210> 7
<211> 348
<212> ADN
<213> Neisseria meningitidis (group B)

<400> 7
atgccagcca gaaactcaaa agcatggtgg aacactacta tgacaaacaa cgttgatttg 60
aaggttcccg caaaacgcta tttcacgctg gacgagttgt gcggactgtt gcaaatcagc 120
ccctatggtt ttgcgcaatg gcagcatgat tatggtgtgg ttgtcggtta cggcggcgaa 180
cgctacaccc gtttggatgt ggtgaaactg ttgaaattgc agagcacgtt tgcaccgtat 240
gcagaaggtg cggagtcggg ttcggacggc aaccgtccgg ttacgcttca ggaaatcgga 300
gacggtctga aagagctgtt ggcggatttg gataaggaat tgtgctga             348


<210> 8
<211> 29
<212> ADN
<213> Artificial Sequence

<220>
<223> Artificial Sequence Description:Oligo 1573

<400> 8
ttccatggta gataaaagaa tggctttag                                          29


<210> 9
<211> 27
<212> ADN
<213> Artificial Sequence

<220>
<223> Artificial Sequence Description:Oligo 6795

<400> 9
aactgcaggc ttgtaaaccg ttttgtg                                            27